# EUROPEAN PATENT SPECIFICATION

(11) **EP 2 735 608 B1**
(45) Date of publication and mention of the grant of the patent: **29.07.2015**
(21) Application number: 13165156.4
(22) Date of filing: 24.04.2013
(51) Int. Cl.: C12N 5/00, B01J 13/04, A61K 9/50

(54) **Core-shell composite having poly-vinylalcohol and alginate and method for fabricating the same**
Kernschalenverbundstoff mit Polyvinylalkohol und Alginat und Herstellungsverfahren dafür
Composite de c'ur-écorce ayant du poly-vinylalcool et de l'alginate et son procédé de fabrication

(30) Priority: 26.11.2012 KR 20120134310
(43) Date of publication of application: 28.05.2014
(73) Proprietor: Korea Institute of Science and Technology, Seoul 136-791 (KR)
(72) Inventor: Bae, Hyokwan, 138-782 Seoul (KR); Lee, Seockheon, 136-791 Seoul (KR); Chung, Yunchul, 137-073 Seoul (KR); Jung, Jin-Young, 706-833 Daegu (KR); Yang, Hee Jeong, 136-130 Seoul (KR)
(74) Representative: advotec.

(56) References cited:
- WO-A1-02/057009
- WO-A2-2004/069135
- US-A1- 2011 263 018

## Description

### [Technical Field]

The present disclosure relates to a core-shell structure composite carrier based on poly(vinyl alcohol) (PVA) and alginate and a method for preparing the same. More particularly, the present disclosure relates to a core-shell structure composite carrier based on PVA and alginate, which has excellent physical properties by using PVA and alginate and is applicable to various hydrotreatment process by immobilizing a specific reactant to each of the core region and shell region depending on the material to be treated, as well as to a method for preparing the same.

### [Background Art]

Microorganism immobilization has been used widely in food fermentation processes and environmental treatment processes. Since microorganisms and enzymes used in food treatment processes require a lot of time and cost for their culture, immobilization has been used for the purpose of easy and efficient recovery. Microorganism immobilization used in environmental treatment processes is mainly for the removal of toxic organic substances and treatment of nitrogen. This is because microorganisms decomposing toxic substances tend to undergo degradation of activity due to the toxicity so that they may be lost during a continuous process. Nitrosomonas and Nitrobacter that belong to nitrifying microorganisms are autotrophs using carbon dioxide as a carbon source, and thus show a lower growth rate as compared to organic microorganisms and are not maintained at a constant concentration during a continuous process. To solve this, immobilization methods have been used to prevent the loss of microorganisms and to maintain a high concentration of microorganisms.

Immobilization methods used in the field of environmental purification are classified broadly into two types. The first type is self-immobilization in which microorganisms are attached to and grown on a supporting matrix by Extracellular Polymeric Substances (EPS) that are viscous polymeric substances secreted by microorganisms. Such self-immobilization is disadvantageous in that it requires a specific condition under which microorganisms form a bio-membrane or requires a certain time for forming a bio-membrane. As a support, a tube-like synthetic polymeric material and sponge-like synthetic polymeric material are used frequently. The second type is encapsulated immobilization in which a mixed solution containing a polymeric material such as alginate mixed homogeneously with microorganism culture is reacted with a crosslinking agent, such as calcium chloride, to form beads, and then microorganisms are highly integrated in the beads at a high concentration.

Ca-alginate beads used frequently in the encapsulated immobilization method according to the related art (Korean Patent Publication No. 919508) have low mechanical strength and undergo cleavage of their binding structure due to phosphate ions, and thus are limited in practical use. In addition, Santos et al. have suggested that a core-shell dual structure is used to provide core-shell structure beads (V.A.P.M dos Santos et al., Modeling and evaluation of an integrated nitrogen removal system with microorganisms co-immobilization in double-layer gel beads, Biotechnology Progress, 1996, 12, 240-248). Since the dual structure suggested herein includes natural polymeric beads based on alginate and κ-carrageenan, it shows low mechanical strength and is not amenable to practical processes. In addition, its application is limited in a conventional nitrification and denitrification process.

### [References of the Related Art]

### [Patent Document]

(Patent Document 1) Korean Patent Publication No. 919508

### [Non-patent Document]

(Non-patent Document 1) V.A.P.M dos Santos et al., Modeling and evaluation of an integrated nitrogen removal system with microorganisms co-immobilization in double-layer gel beads, Biotechnology Progress, 1996, 12, 240-248

### [Disclosure]

### [Technical Problem]

A technical problem to be solved by the present disclosure is to provide a core-shell structure composite carrier based on PVA and alginate, which has excellent physical properties by using PVA and alginate and is.applicable to various hydrotreatment process by immobilizing a specific reactant to each of the core region and shell region depending on the material to be treated, as well as a method for preparing the same.

### [Technical Solution]

In one general aspect, there is provided a method for preparing a core-shell structure composite carrier based on poly(vinyl alcohol) (PVA) and alginate, including: providing a mixed solution for a core containing PVA, alginate and a second reactant; introducing the mixed solution for a core to a boric acid solution containing calcium chloride to form beads of a core region to which the second reactant is immobilized; immersing the beads of a core region into a boric acid solution containing calcium chloride to form a crosslinking agent on the beads of a core region; and introducing the beads of a core region having the crosslinking agent to a mixed solution for a shell to form beads of a shell region on the surface of the beads of a core region, wherein the mixed solution for a shell includes PVA, alginate and a first reactant.

The mixed solution for a core may be a mixture of a PVA-alginate mixed solution in combination with a second reactant-containing mixed solution, and the PVA-alginate mixed solution may be a solution containing 10-20 w/v% of PVA in combination with 1-5 w/v% of sodium alginate dissolved in distilled water. The second reactant-containing mixed solution may be a solution containing the second reactant dissolved in distilled water. In addition, the mixed solution for a shell may be a mixture of a PVA-alginate mixed solution in combination with a first reactant-containing mixed solution, and the PVA-alginate mixed solution may be a solution containing 10-20 w/v% of PVA in combination with 1-5 w/v% of sodium alginate dissolved in distilled water. Further, the first reactant-containing mixed solution may be a solution containing the first reactant dissolved in distilled water.

The first reactant may be an aerobic nitrifying microorganism, and the second reactant may be an anaerobic ammonia oxidizing microorganism. In a variant, the first reactant may be zero-valent iron by which nitrate is converted into ammonia, and the second reactant may be a solid adsorbent on which ammonia is adsorbed. In another variant, the first reactant may be an anaerobic microorganism for removing a toxic organic substance, and the second reactant may be an anaerobic microorganism.

After the forming the beads of a core region, and after the forming the beads of a shell region, the finished beads of a core region or those of a shell region may be immersed into a phosphoric acid solution to reinforce the mechanical strength of the beads. At that time, alginate is decomposed structurally but the synthetic polymer, PVA, has increased strength.

In the forming the beads of a core region, the mixed solution for a core may be introduced through any one selected from a needle, tube and hopper to control the size of the beads of a core region. In addition, the beads of a shell region may have a controlled thickness by controlling the time during which the beads of a core region is immersed into a boric acid solution. Further, the beads of a shell region may have a controlled thickness by controlling the time during which the beads of a core region having the crosslinking agent is introduced to the mixed solution for a shell.

The core-shell structure composite carrier based on PVA and alginate includes beads of a core region and beads of a shell region formed on the surface of the beads of a core region, wherein a first reactant carrying out a first unit reaction is immobilized to the beads of a shell region, a second reactant carrying out a second unit reaction is immobilized to the beads of a core region, the first unit reaction and the second unit reaction are independent from each other and are time series continuous reactions, and the beads of a core region and the beads of a shell region include PVA and alginate.

### [Advantageous Effects]

The core-shell structure composite carrier based on PVA and alginate according to the embodiments of the present disclosure and the method for preparing the same provide the following effects.

Since the core-shell structure composite carrier has a dual structure having a core region and a shell region, and each region is formed from beads based on PVA and alginate, it is possible to improve the mechanical strength of the composite carrier. In addition, when the core region and the shell region are provided with multiple reactants, it is possible to carry out complex hydrotreatment by using a single composite carrier possessing multiple reactions.

### [Description of Drawings]

The above and other aspects, features and advantages of the disclosed exemplary embodiments will be more apparent from the following detailed description taken in conjunction with the accompanying drawings in which:
Fig. 1 is a flow chart illustrating a method for preparing a core-shell structure composite carrier based on poly(vinyl alcohol) (PVA) and alginate according to an embodiment of the present disclosure;
Fig. 2 is a photographic image of finished beads of a core region;
Fig. 3 is a schematic view illustrating a needle, tube and hopper through which a mixed solution for a core is introduced;
Fig. 4 is a graph illustrating the thickness of beads of a shell region as a function of immersing time in a saturated boric acid solution;
Fig. 5 is a graph illustrating the thickness of beads of a shell region as a function of immersing time in a mixed solution for a shell;
Fig. 6 is a photographic image of the core-shell structure composite carrier obtained in accordance with an embodiment of the present disclosure; and
Fig. 7 is a graph illustrating the nitrogen removal efficiency of the composite carrier according to an embodiment of the present disclosure.

### [Best Mode]

Exemplary embodiments now will be described more fully hereinafter with reference to the accompanying drawings, in which exemplary embodiments are shown.

In one aspect, there is provided a core-shell structure composite carrier based on poly(vinyl alcohol) (PVA) and alginate. Both the core region and the shell region are formed from beads of PVA and alginate. After a spherical core region is formed, a shell region is formed on the surface of the core region. Since the composite carrier has a dual structure of a core region and a shell region and uses a synthetic polymeric material, PVA, it ensures high mechanical strength.

While the composite carrier according to the related art carries out a specific unit reaction, such as contaminant adsorption or microorganism decomposition, the composite carrier according to the present disclosure is **characterized in that** it carries out a plurality of unit reactions through a single composite carrier. In other words, a first unit reaction is performed by a shell region and a second unit reaction is performed by a core region, wherein the first unit reaction and the second unit reaction refer to time series reactions of a sewage/waste water treatment process. For example, nitrification and denitrification in a sewage/waste water treatment process have been carried out sequentially in separate reactors. However, the composite carrier according to the present disclosure allows for the shell region and the core region to carry out nitrification (the first unit reaction) and autotrophic denitrification (the second unit reaction), respectively.

Depending on the contaminant to be treated, the first unit reaction and the second unit reaction are defined, and the optimum reactant for the first unit reaction and the optimum reactant for the second unit reaction are immobilized to the shell region and the core region, respectively. As mentioned above, when the first unit reaction is nitrification and the second unit reaction is autotrophic denitrification, aerobic nitrifying microorganisms are immobilized to the shell region as a reactant, and anaerobic ammonia oxidizing microorganisms are immobilized to the core region.

In addition, when nitrate (NO₃⁻) is to be treated, the first unit reaction carried out by the shell region may be defined as conversion of nitrate (NO₃⁻) into ammonia and the second unit reaction may be defined as adsorption of ammonia. In this case, zero-valent iron is immobilized to the shell region and an adsorbent for ammonia, such as zeolite, may be immobilized to the core region.

The composite carrier may be applied to waste water containing a toxic organic substance. In this case, anaerobic microorganisms for removing the toxic organic substance, such as *Dehalococcoides*, may be immobilized to the shell region, and a general anaerobic microbial population for purifying waste water from which the toxic organic substance is removed may be immobilized to the core region.

As described above, the composite carrier according to the present disclosure performs time-series reactions of the first unit reaction carried out by the shell region and the second unit reaction carried out by the core region, wherein the shell region is distinguished spatially from the core region, and thus the first unit reaction and the second unit reaction are carried out independently from each other.

In addition, dissolved oxygen characteristics of the core region and those of the shell region may be varied by controlling the thickness of the shell region. By using this, it is possible to immobilize aerobic nitrifying microorganisms to the shell region having a relatively large amount of dissolved oxygen, and anaerobic microorganisms to the core region having a relatively small amount of dissolved oxygen. When applying the composite carrier according to the present disclosure to sewage/waste water treatment, an increase in thickness of the shell region causes a gradient in dissolved oxygen concentration depending on the distance between the shell region and the core region. By using this, it is possible to provide aerobic microorganisms and anaerobic microorganisms to the shell region and the core region, respectively.

Hereinafter, the method for preparing a core-shell structure composite carrier based on PVA and alginate according to an embodiment of the present disclosure and the composite carrier obtained thereby will be explained in detail with reference to the accompanying drawings.

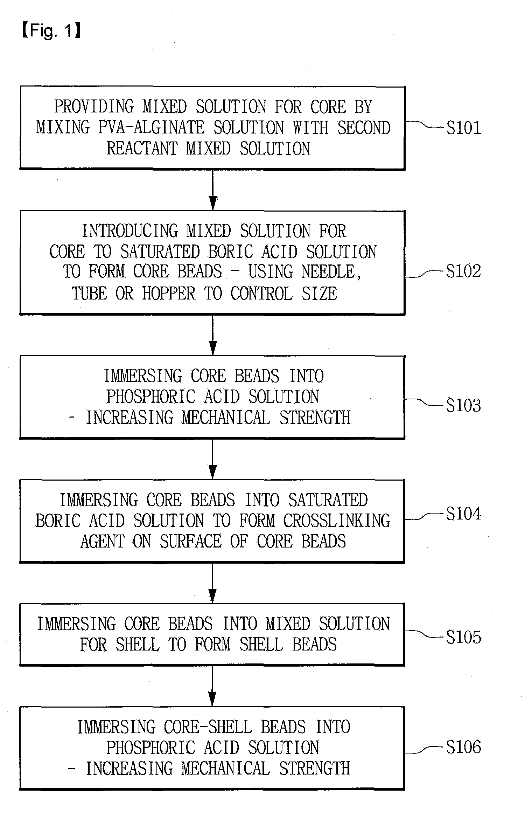

Referring to Fig. 1, a PVA-alginate mixed solution and a second reactant-containing mixed solution are provided (S101). The PVA-alginate mixed solution is a solution containing 10-20 w/v% of PVA in combination with 1-5 w/v% of sodium alginate dissolved in distilled water. The second reactant-containing mixed solution is a solution containing the second reactant. The second reactant is a material to be immobilized to a core region, i.e., PVA-alginate beads of a core region, and carries out the second unit reaction as mentioned above. The solvent in which the second reactant is dissolved is determined by the characteristics of the second reactant. For example, when the second reactant is microbial, the second reactant-containing mixed solution may be a mixture of microorganisms with sludge. In addition, the second reactant may be varied with the subject to be treated. In the case of autotrophic denitrification, anaerobic ammonia oxidizing microorganisms may be the second reactant. In the case of ammonia adsorption, an adsorbent such as zeolite may be the second reactant.

After providing the PVA-alginate mixed solution and the second reactant-containing mixed solution, the two solutions are mixed and agitated with each other. The resultant mixture is also referred to as 'mixed solution for a core' hereinafter. For reference, when the second reactant is microbial, the mixed solution for a core may be maintained at a temperature of 35-40°C.

Then, the mixed solution for a core is introduced to a saturated boric acid (H₃BO₃) solution containing 0.5-1 w/v% of calcium chloride (CaCl₂) (S102). Calcium chloride and a saturated boric acid function as crosslinking agents by which the mixed solution for a core is converted into beads. Particularly, calcium chloride functions as a crosslinking agent for alginate and a saturated boric acid functions as a crosslinking agent for PVA. Herein, to form spherical beads, the mixed solution for a core is introduced to a saturated boric acid solution through a needle, tube or hopper. According to the use of a needle, tube or hopper, it is possible to introduce the mixed solution for a core to a saturated boric acid solution in the form of spheres.

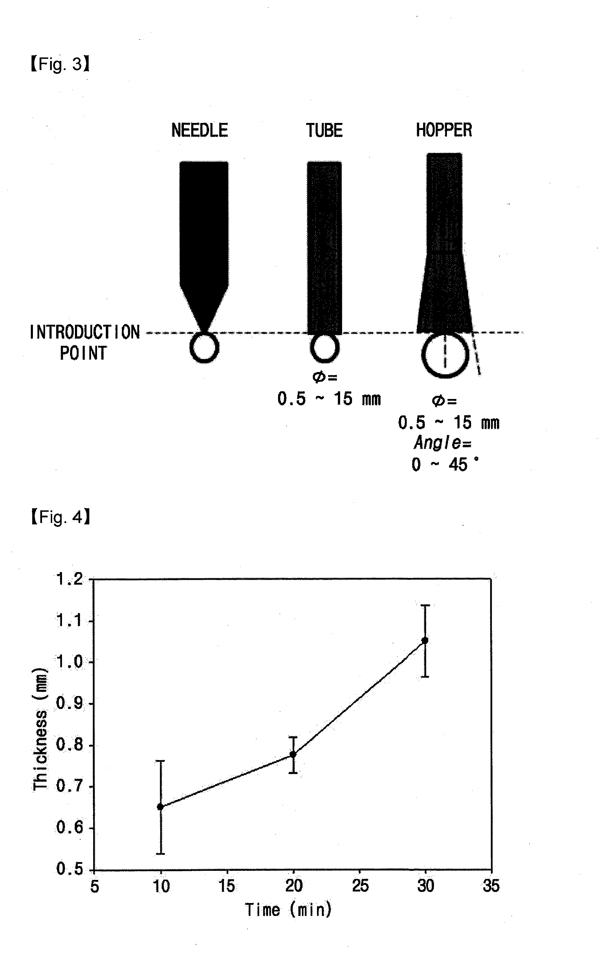

It is possible to form beads by introducing the mixed solution for a core to a saturated boric acid solution. The resultant beads correspond to the beads of a core region in the composite carrier according to the present disclosure. In addition, the beads of a core region may be controlled in size, if desired. Controlling the bead size may be performed by using a needle, tube or hoper. When using a needle, beads with a size of 3-4 mm may be formed. When using a tube with a size of 0.5-1.5 mm, beads with a size of 0.5-15 mm may be formed. In addition, use of a hopper may provide beads having a larger diameter (see Fig. 3). Meanwhile, to prevent agglomeration during the formation of the beads from the mixed solution for a core, the mixed solution for a core may be introduced while agitating a saturated boric acid solution.

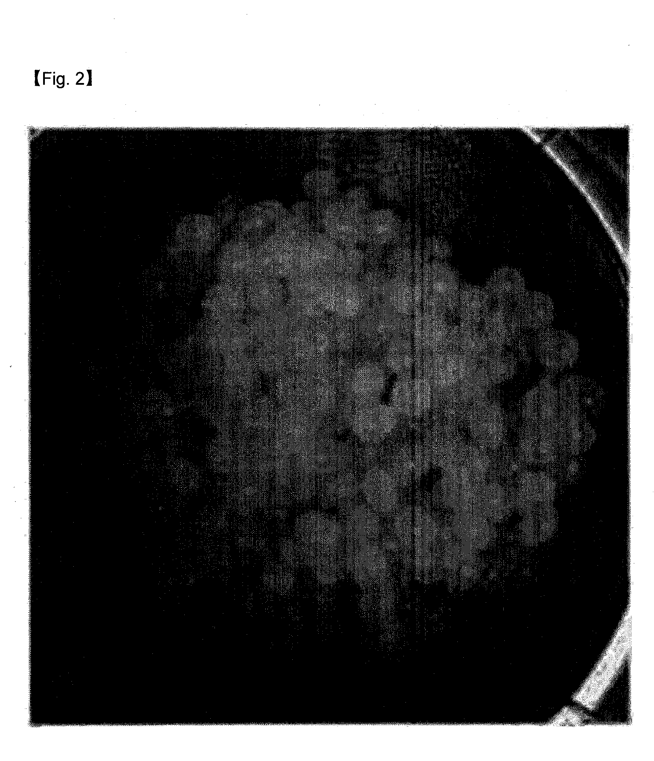

After the beads of a core region are finished, the beads of a core region are immersed into 0.5-1M phosphoric acid solution to reinforce the mechanical strength of the beads (S103). Fig. 2 is a photographic image of the beads of a core region.

After completing the formation of the beads of a core region as described above, beads of a shell region are formed. The beads of a shell region are formed on the surface of the beads of a core region.

First, the beads of a core region are immersed into a saturated boric acid (H₃BO₃) solution containing 0.5-1 w/v% of calcium chloride (CaCl₂) to form a crosslinking agent on the surface of the beads of a core region (S104). As mentioned above, calcium chloride functions as a crosslinking agent for alginate and a saturated boric acid functions as a crosslinking agent for PVA. As the beads of a core region are immersed into a saturated boric acid solution, the crosslinking agents are formed on the surface of the beads of a core region. Herein, the amount of crosslinking agents is in proportion to the immersion time. The amount of crosslinking agents is also in proportion to the thickness of beads. In other words, as immersion time increases, the thickness of the beads of a shell region increases. When the thickness of the beads of a shell region increases above a predetermined value, it is possible to realize a gradient in dissolved oxygen concentration depending on the distance between the shell region and the core region. By using this, it is possible to provide aerobic microorganisms and anaerobic microorganisms to the shell region and the core region, respectively. Meanwhile, it can be seen from Fig. 4 that the thickness of the beads of a shell region increases in proportion to the immersion time.

Next, the beads of a core region immersed in a saturated boric acid solution are introduced to a mixed solution for a shell to form beads of a shell region on the surface of the beads of a core region (S105). The mixed solution for a shell is a mixture of a PVA-alginate mixed solution in combination with a first reactant-containing mixed solution, and the PVA-alginate mixed solution is a solution containing 10-20 w/v% of PVA in combination with 1-5 w/v% of sodium alginate dissolved in distilled water. In addition, the first reactant-containing mixed solution is a solution containing the first reactant. The first reactant is a material to be immobilized to the shell region, i.e. PVA-alginate beads of a shell region and carries out the first unit reaction as mentioned above. Similarly to the second reactant, the first reactant may be varied with the subject to be treated. In the case of nitrification, aerobic nitrifying microorganisms may be applied as the first reactant. In the case of conversion of nitrate into ammonia, zero-valent iron may be applied as the first reactant.

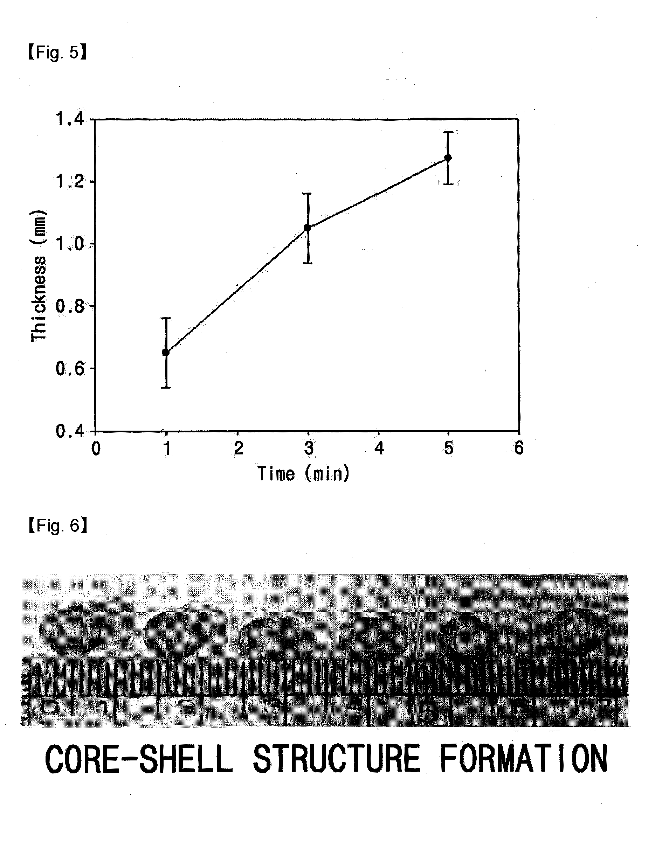

As the beads of a core region are introduced to the mixed solution for a shell after providing the crosslinking agents to the surface of the beads of a core region, beads including PVA and alginate, i.e., beads for a shell region are formed on the surface of the beads of a core region. Herein, the thickness of the beads of a shell region varies also with the immersion time in the mixed solution for a shell. It can be seen from Fig. 5 that the thickness of the beads of a shell region increases in proportion to the immersion time in the mixed solution for a shell.

After the completion of the formation of the beads of a shell region, i.e., the formation of the core-shell structure having the beads of a core region and those of a shell region, the core-shell structure beads are immersed into 0.5-1M phosphoric acid solution to reinforce the mechanical strength of the beads (S106). In this manner, the composite carrier according to the present disclosure is finished. Fig. 6 is a photographic image of the core-shell structure composite carrier obtained in accordance with an embodiment of the present disclosure.

Meanwhile, the procedure from the step of immersing the beads of a core region into a saturated boric acid solution to the step of immersing the core-shell structure beads into a phosphoric acid solution is performed repeatedly in order to increase the thickness of a shell region.

Hereinabove, the core-shell structure composite carrier based on PVA and alginate according to an embodiment and the method for preparing the same are described. The composite carrier obtained according to an embodiment of the present disclosure will be characterized hereinafter.

### <Test Example: Nitrogen Removal Efficiency>

A core-shell structure composite carrier in which anaerobic ammonium oxidizing microorganisms are immobilized to the beads of a core region and aerobic nitrifying microorganisms are immobilized to the beads of a shell region is provided and applied to a sewage/waste water treatment process.

In this example, simultaneous nitrification/autotrophic denitrification is realized by the core-shell structure composite carrier. Particularly, in a shell region capable of diffusion of dissolved oxygen, nitrifying microorganisms convert ammonia (NH₄⁺) into nitrite (NO₂⁻). In addition, in a core region maintaining an anaerobic state through the inhibition of diffusion of dissolved oxygen caused by the shell region, nitrous acid and ammonia diffused from the exterior are converted into nitrogen gas. In this manner, the core-shell structure composite carrier realizes nitrification and autotrophic denitrification at the same time.

Nitrifying microorganisms are used in the same form as in active sludge of the existing sewage treatment plant. Anaerobic ammonium oxidizing bacteria is obtained by highly concentrated culture of active sludge in a selective medium in the encapsulated immobilized form to PVA/alginate beads. The selective medium maintains (NH₄)₂SO₄ and NaNO₂ that are main energy sources for anaerobic ammonium oxidizing microorganisms at the same concentration of 50 mg/L in terms of nitrogen, and is prepared from NaCO₃-C 350 mg/L, KH₂PO₄-P 6 mg/L, MgSO₄•H₂O-Mg 12 mg/L, CaCl₂•2H₂O-Ca 48 mg/L, trace element I solution (EDTA 5 g/L and FeSO₄•7H₂O 5 g/L) 1ml/L, trace element II solution (ZnSO₄•7H₂O 0.43 g/L, CoCl₂•6H₂O 0.24 g/L, MnCl₂•4H₂O 0.99 g/L, CuSO₄•5H₂O 0.25 g/L, Na₂MoO₄•2H₂O 0.22 g/L, NiCl₂•6H₂O 0.19 g/L, Na₂SeO₄•10H₂O 0.21 g/L, H₃BO₃ 0.014 g/L) 1 ml/L. The total volume of the reactor is set to 500 mL and the reactor is operated under the condition of oxygen feed of 1 L/min, at 35°C where nitrification and anaerobic ammonium oxidization are optimized, for a hydraulic retention time of 4.17 hours. The core-shell beads are packed at a ratio of 8%. The core beads have a size of 3-4 mm and the shell layer has an average thickness of 3.2 mm.

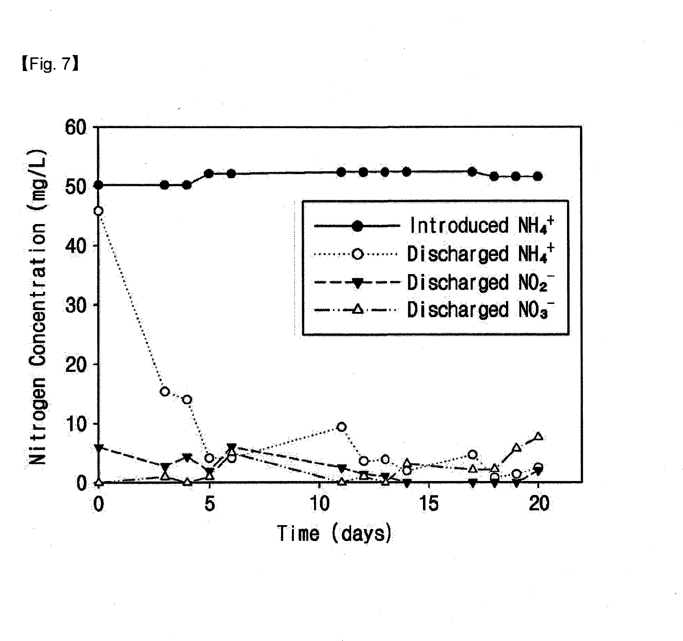

After the test, as can be seen from Fig. 7, the nitrifying microorganisms show an increase in activity for the first five days, resulting in a decrease in amount of discharged ammonia nitrogen. After the fifth day, the nitrifying microorganisms and the anaerobic ammonia oxidizing microorganisms carry out a continuous reaction, thereby providing an excellent nitrogen removal efficiency of 84.7±7.8%.

## Claims

1. A method for preparing a core-shell structure composite carrier based on poly(vinyl alcohol) (PVA) and alginate, comprising:
providing a mixed solution for a core containing PVA, alginate and a second reactant;
introducing the mixed solution for a core to a boric acid solution containing calcium chloride to form beads of a core region to which the second reactant is immobilized;
immersing the beads of a core region into a boric acid solution containing calcium chloride to form a crosslinking agent on the beads of a core region; and
introducing the beads of a core region having the crosslinking agent to a mixed solution for a shell to form beads of a shell region on the surface of the beads of a core region,
wherein the mixed solution for a shell comprises PVA, alginate and a first reactant.

2. The method for preparing a core-shell structure composite carrier based on PVA and alginate according to claim 1, wherein the mixed solution for a core is a mixture of a PVA-alginate mixed solution in combination with a second reactant-containing mixed solution, the PVA-alginate mixed solution is a solution containing 10-20 w/v% of PVA in combination with 1-5 w/v% of sodium alginate dissolved in distilled water, and the second reactant-containing mixed solution is a solution containing the second reactant.

3. The method for preparing a core-shell structure composite carrier based on PVA and alginate according to claim 1, wherein the mixed solution for a shell is a mixture of a PVA-alginate mixed solution in combination with a first reactant-containing mixed solution, the PVA-alginate mixed solution is a solution containing 10-20 w/v% of PVA in combination with 1-5 w/v% of sodium alginate dissolved in distilled water, and the first reactant-containing mixed solution is a solution containing the first reactant.

4. The method for preparing a core-shell structure composite carrier based on PVA and alginate according to one of claims 1 to 3, wherein the first reactant is an aerobic nitrifying microorganism, and the second reactant is an anaerobic ammonia oxidizing microorganism.

5. The method for preparing a core-shell structure composite carrier based on PVA and alginate according to one of claims 1 to 3, wherein the first reactant is zero-valent iron by which nitrate is converted into ammonia, and the second reactant is a solid adsorbent on which ammonia is adsorbed.

6. The method for preparing a core-shell structure composite carrier based on PVA and alginate according to one of claims 1 to 3, wherein the first reactant is an anaerobic microorganism for removing a toxic organic substance, and the second reactant is an anaerobic microorganism.

7. The method for preparing a core-shell structure composite carrier based on PVA and alginate according to one of claims 1 to 6, which further comprises, after said forming the beads of a core region, and after said forming the beads of a shell region, immersing the finished beads of a core region or those of a shell region into a phosphoric acid solution to reinforce the mechanical strength of the beads.

8. The method for preparing a core-shell structure composite carrier based on PVA and alginate according to one of claims 1 to 7, wherein the mixed solution for a core is introduced through any one selected from a needle, tube and hopper to control the size of the beads of a core region, in said forming the beads of a core region.

9. The method for preparing a core-shell structure composite carrier based on PVA and alginate according to one of claims 1 to 8, wherein the time during which the beads of a core region is immersed into a boric acid solution is controlled to control the thickness of the beads of a shell region, in said immersing the beads of a core region into a boric acid solution containing calcium chloride to form a crosslinking agent on the beads of a core region.

10. The method for preparing a core-shell structure composite carrier based on PVA and alginate according to one of claims 1 to 8, wherein the time during which the beads of a core region having the crosslinking agent is introduced to the mixed solution for a shell is controlled to control the thickness of the beads of a shell region, in said introducing the beads of a core region having the crosslinking agent to a mixed solution for a shell to form beads of a shell region on the surface of the beads of a core region.

11. A core-shell structure composite carrier based on poly(vinyl alcohol) (PVA) and alginate, comprising beads of a core region and beads of a shell region formed on the surface of the beads of a core region, wherein a first reactant carrying out a first unit reaction is immobilized to the beads of a shell region, a second reactant carrying out a second unit reaction is immobilized to the beads of a core region, the first unit reaction and the second unit reaction are independent from each other and are time series continuous reactions, and the beads of a core region and the beads of a shell region comprise PVA and alginate.

12. The core-shell structure composite carrier based on PVA and alginate according to claim 11, wherein the first reactant is an aerobic nitrifying microorganism, and the second reactant is an anaerobic ammonia oxidizing microorganism.

13. The core-shell structure composite carrier based on PVA and alginate according to claim 11, wherein the first reactant is zero-valent iron by which nitrate is converted into ammonia, and the second reactant is a solid adsorbent on which ammonia is adsorbed.

14. The core-shell structure composite carrier based on PVA and alginate according to claim 11, wherein the first reactant is an anaerobic microorganism for removing a toxic organic substance, and the second reactant is an anaerobic microorganism.

## Patentansprüche

1. Verfahren zur Herstellung eines Verbundträgers mit einer Kern-Schale-Struktur auf Basis von Polyvinylalkohol (PVA) und Alginat, umfassend:
Bereitstellen einer PVA, Alginat und einen zweiten Reaktanten enthaltenden Mischlösung für einen Kern;
Einbringen der Mischlösung für einen Kern in eine Calciumchlorid enthaltene Borsäurelösung zur Bildung von Kügelchen eines Kernbereichs, an denen der zweite Reaktant immobilisiert ist;
Eintauchen der Kügelchen eines Kernbereichs in eine Calciumchlorid enthaltende Borsäurelösung zur Bildung eines Vernetzungsmittels auf den Kügelchen eines Kernbereichs; und
Einbringen der das Vernetzungsmittel aufweisenden Kügelchen eines Kernbereichs in eine Mischlösung für eine Schale zur Bildung von Kügelchen eines Schalenbereichs auf der Oberfläche der Kügelchen eines Kernbereichs,
wobei die Mischlösung für eine Schale PVA, Alginat und einen ersten Reaktanten umfasst.

2. Verfahren zur Herstellung eines Verbundträgers mit einer Kern-Schale-Struktur auf Basis von PVA und Alginat nach Anspruch 1, wobei die Mischlösung für einen Kern eine Mischung aus einer PVA-Alginat-Mischlösung in Kombination mit einer einen zweiten Reaktanten enthaltenden Mischlösung ist, wobei die PVA-Alginat-Mischlösung eine Lösung ist, die 10-20 w/v% PVA in Kombination mit in destilliertem Wasser gelöstem 1-5 w/v% Natriumalginat enthält, und die einen zweiten Reaktanten enthaltende Mischlösung eine Lösung ist, die den zweiten Reaktanten enthält.

3. Verfahren zur Herstellung eines Verbundträgers mit Kern-Schale-Struktur auf Basis von PVA und Alginat nach Anspruch 1, wobei die Mischlösung für eine Schale eine Mischung aus einer PVA-Alginat-Mischlösung in Kombination mit einer einen ersten Reaktanten enthaltenden Mischlösung ist, wobei die PVA-Alginat-Mischlösung eine Lösung ist, die 10-20 w/v% PVA in Kombination mit in destilliertem Wasser gelöstem 1-5 w/v% Natriumalginat enthält, und die einen ersten Reaktanten enthaltende Mischlösung eine Lösung ist, die den ersten Reaktanten enthält.

4. Verfahren zur Herstellung eines Verbundträgers mit Kern-Schale-Struktur auf Basis von PVA und Alginat nach einem der Ansprüche 1 bis 3, wobei der erste Reaktant ein aerober nitrifizierender Mikroorganismus ist und der zweite Reaktant ein anaerober Ammoniak oxidierender Mikroorganismus ist.

5. Verfahren zur Herstellung eines Verbundträgers mit Kern-Schale-Struktur auf Basis von PVA und Alginat nach einem der Ansprüche 1 bis 3, wobei der erste Reaktant nullwertiges Eisen ist, durch das Nitrat in Ammoniak umgewandelt wird, und der zweite Reaktant ein festes Adsorbens ist, an dem Ammoniak adsorbiert wird.

6. Verfahren zur Herstellung eines Verbundträgers mit Kern-Schale-Struktur auf Basis von PVA und Alginat nach einem der Ansprüche 1 bis 3, wobei der erste Reaktant ein anaerober Mikroorganismus zur Beseitigung einer toxischen organischen Substanz ist und der zweite Reaktant ein anaerober Mikroorganismus ist.

7. Verfahren zur Herstellung eines Verbundträgers mit Kern-Schale-Struktur auf Basis von PVA und Alginat nach einem der Ansprüche 1 bis 6, das nach der Ausbildung der Kügelchen eines Kernbereichs und nach der Ausbildung der Kügelchen eines Schalenbereichs weiterhin das Eintauchen der fertigen Kügelchen eines Kernbereichs oder der fertigen Kügelchen eines Schalenbereichs in eine Phosphorsäurelösung zur Verstärkung der mechanischen Festigkeit der Kügelchen umfasst.

8. Verfahren zur Herstellung eines Verbundträgers mit Kern-Schale-Struktur auf Basis von PVA und Alginat nach einem der Ansprüche 1 bis 7, wobei die Mischlösung für einen Kern bei der Bildung der Kügelchen eines Kernbereichs zur Steuerung der Größe der Kügelchen eines Kernbereichs wahlweise durch eine Nadel, ein Röhrchen oder einen Trichter zugeführt wird.

9. Verfahren zur Herstellung eines Verbundträgers mit Kern-Schale-Struktur auf Basis von PVA und Alginat nach einem der Ansprüche 1 bis 8, wobei bei dem Eintauchen der Kügelchen eines Kernbereichs in eine Calciumchlorid enthaltende Borsäurelösung zur Bildung eines Vernetzungsmittels auf den Kügelchen eines Kernbereichs die Dauer des Eintauchens der Kügelchen eines Kernbereichs in eine Borsäurelösung zur Steuerung der Dicke der Kügelchen eines Schalenbereichs gesteuert wird.

10. Verfahren zur Herstellung eines Verbundträgers mit Kern-Schale-Struktur auf Basis von PVA und Alginat nach einem der Ansprüche 1 bis 8, wobei bei dem Einbringen der das Vernetzungsmittel aufweisenden Kügelchen eines Kernbereichs in eine Mischlösung für eine Schale zur Bildung von Kügelchen eines Schalenbereichs auf der Oberfläche der Kügelchen eines Kernbereichs die Dauer des Einbringens der das Vernetzungsmittel aufweisenden Kügelchen eines Kernbereichs in die Mischlösung für eine Schale zur Steuerung der Dicke der Kügelchen eines Schalenbereichs gesteuert wird.

11. Verbundträger mit Kern-Schale-Struktur auf Basis von Polyvinylalkohol (PVA) und Alginat, umfassend Kügelchen eines Kernbereichs und Kügelchen eines Schalenbereichs, die auf der Oberfläche der Kügelchen eines Kernbereichs ausgebildet sind, wobei ein erster Reaktant, der eine erste Einzelreaktion ausführt, auf den Kügelchen eines Schalenbereichs immobilisiert ist, ein zweiter Reaktant, der eine zweite Einzelreaktion ausführt, auf den Kügelchen eines Kernbereichs immobilisiert ist, wobei die erste Einzelreaktion und die zweite Einzelreaktion voneinander unabhängig sind und zeitlich aufeinanderfolgende kontinuierliche Reaktionen sind und die Kügelchen eines Kernbereichs und die Kügelchen eines Schalenbereichs PVA und Alginat umfassen.

12. Verbundträger mit Kern-Schale-Struktur auf Basis von PVA und Alginat nach Anspruch 11, wobei der erste Reaktant ein aerober nitrifizierender Mikroorganismus ist und der zweite Reaktant ein anaerober Ammoniak oxidierender Mikroorganismus ist.

13. Verbundträger mit Kern-Schale-Struktur auf Basis von PVA und Alginat nach Anspruch 11, wobei der erste Reaktant nullwertiges Eisen ist, durch das Nitrat in Ammoniak umgewandelt wird, und der zweite Reaktant ein festes Adsorbens ist, an dem Ammoniak adsorbiert wird.

14. Verbundträger mit Kern-Schale-Struktur auf Basis von PVA und Alginat nach Anspruch 11, wobei der erste Reaktant ein anaerober Mikroorganismus zur Beseitigung einer toxischen organischen Substanz ist und der zweite Reaktant ein anaerober Mikroorganismus ist.

## Revendications

1. Procédé de préparation d'un support composite ayant une structure noyau-enveloppe à base d'alcool polyvinylique (APV) et d'alginate, comprenant :
fournir une solution mixte pour un noyau contenant de l'APV, de l'alginate et un deuxième réactif ;
introduire la solution mixte pour un noyau dans une solution d'acide borique contenant du chlorure de calcium afin de former des billes d'une région de noyau sur lesquelles le deuxième réactif est immobilisé ;
immerger les billes d'une région de noyau dans une solution d'acide borique contenant du chlorure de calcium afin de former un agent de réticulation sur les billes d'une région de noyau ; et
introduire les billes d'une région de noyau ayant l'agent de réticulation dans une solution mixte pour une enveloppe afin de former des billes d'une région d'enveloppe sur la surface des billes d'une région de noyau,
dans lequel la solution mixte pour une enveloppe comprend l'APV, l'alginate et un premier réactif.

2. Procédé de préparation d'un support composite ayant une structure noyau-enveloppe à base d'APV et d'alginate selon la revendication 1, dans lequel la solution mixte pour un noyau est un mélange d'une solution mixte APV-alginate en combinaison avec une solution mixte contenant un deuxième réactif, la solution mixte APV-alginate est une solution contenant 10-20 w/v% de APV en combinaison avec 1-5 w/v% d'alginate de sodium dissous dans l'eau distillée, et la solution mixte contenant un deuxième réactif est une solution qui contient le deuxième réactif.

3. Procédé de préparation d'un support composite ayant une structure noyau-enveloppe à base d'APV et d'alginate selon la revendication 1, dans lequel la solution mixte pour une enveloppe est un mélange d'une solution mixte APV-alginate en combinaison avec une solution mixte contenant un premier réactif, la solution mixte APV-alginate est une solution contenant 10-20 w/v% d'APV en combinaison avec 1-5 w/v% d'alginate de sodium dissous dans l'eau distillée, et la solution mixte contenant un premier réactif est une solution qui contient le premier réactif.

4. Procédé de préparation d'un support composite ayant une structure noyau-enveloppe à base d'APV et d'alginate selon l'une quelconque des revendications 1 à 3, dans lequel le premier réactif est un micro-organisme aérobie nitrifiant et le deuxième réactif est un micro-organisme anaérobie oxydant l'ammoniac.

5. Procédé de préparation d'un support composite ayant une structure noyau-enveloppe à base d'APV et d'alginate selon l'une quelconque des revendications 1 à 3, dans lequel le premier réactif est le fer à valence zéro par lequel le nitrate est converti en ammoniac et le deuxième réactif est un adsorbant solide sur lequel l'ammoniac est adsorbé.

6. Procédé de préparation d'un support composite ayant une structure noyau-enveloppe à base d'APV et d'alginate selon l'une quelconque des revendications 1 à 3, dans lequel le premier réactif est un micro-organisme anaérobie pour enlever une substance organique toxique et le deuxième réactif est un micro-organisme anaérobie.

7. Procédé de préparation d'un support composite ayant une structure noyau-enveloppe à base d'APV et d'alginate selon l'une quelconque des revendications 1 à 6 qui comprend en outre, après ladite formation des billes d'une région de noyau et après ladite formation des billes d'une région d'enveloppe, immerger les billes finies d'une région de noyau ou celles d'une région d'enveloppe dans une solution d'acide phosphorique afin de renforcer la résistance mécanique des billes.

8. Procédé de préparation d'un support composite ayant une structure noyau-enveloppe à base d'APV et d'alginate selon l'une quelconque des revendications 1 à 7, dans lequel la solution mixte pour un noyau est introduite au moyen d'un quelconque choisi parmi une aiguille, un tube et un entonnoir afin de contrôler la taille des billes d'une région de noyau lors de la formation des billes d'une région de noyau.

9. Procédé de préparation d'un support composite ayant une structure noyau-enveloppe à base d'APV et d'alginate selon l'une quelconque des revendications 1 à 8, dans lequel la durée pendant laquelle les billes d'une région de noyau sont immergées dans une solution d'acide borique est contrôlée afin de contrôler l'épaisseur des billes d'une région d'enveloppe lors de l'immersion des billes d'une région de noyau dans une solution d'acide borique contenant du chlorure de calcium afin de former un agent de réticulation sur les billes d'une région de noyau.

10. Procédé de préparation d'un support composite ayant une structure noyau-enveloppe à base d'APV et d'alginate selon l'une quelconque des revendications 1 à 8, dans lequel la durée pendant laquelle les billes d'une région de noyau ayant l'agent de réticulation sont introduites dans la solution mixte pour une enveloppe est contrôlée afin de contrôler l'épaisseur des billes d'une région d'enveloppe lors de l'immersion des billes d'une région de noyau ayant l'agent de réticulation dans une solution mixte pour une enveloppe afin de former des billes d'une région d'enveloppe sur la surface des billes d'une région de noyau.

11. Support composite ayant une structure noyau-enveloppe à base d'alcool polyvinylique (APV) et d'alginate comprenant des billes d'une région de noyau et des billes d'une région d'enveloppe formées sur la surfaces des billes d'une région de noyau, dans lequel un premier réactif effectuant une première réaction unique est immobilisé sur les billes d'une région d'enveloppe, un deuxième réactif effectuant une deuxième réaction unique est immobilisé sur les billes d'une région de noyau, la première réaction unique et la deuxième réaction unique sont indépendantes l'une de l'autre et sont des réactions continues successives dans le temps, et les billes d'une région de noyau et les billes d'une région d'enveloppe comprennent l'APV et l'alginate.

12. Support composite ayant une structure noyau-enveloppe à base d'APV et d'alginate selon la revendication 11, dans lequel le premier réactif est un micro-organisme aérobie nitrifiant et le deuxième réactif est un micro-organisme anaérobie oxydant l'ammoniac.

13. Support composite ayant une structure noyau-enveloppe à base d'APV et d'alginate selon la revendication 11, dans lequel le premier réactif est le fer à valence zéro par lequel le nitrate est converti en ammoniac et le deuxième réactif est un adsorbant solide sur lequel l'ammoniac est adsorbé.

14. Support composite ayant une structure noyau-enveloppe à base d'APV et d'alginate selon la revendication 11, dans lequel le premier réactif est un micro-organisme anaérobie pour enlever une substance organique toxique et le deuxième réactif est un micro-organisme anaérobie.
